# EUROPEAN PATENT APPLICATION

(11) **EP 4 796 139 A2**
(43) Date of publication of application: **26.08.2026**
(21) Application number: 26172223.5
(22) Date of filing: 24.11.2021
(51) Int. Cl.: A61M 1/00

(54) **A TUBE**

(30) Priority: 24.11.2020 DK PA202070780
(62) Document number(s) of the earlier application(s) in accordance with Art. 76 EPC: 21819024.7 / 4 251 101
(71) Applicant: Coloplast A/S, 3050 Humlebaek (DK)
(72) Inventor: Radmer, Bo, 3050 Humlebaek (DK)

(57) **Abstract**

A kink-free tube is provided. The tube has a longitudinally extending support, which is unable to be stretched in a longitudinal direction. Furthermore, it is possible to stretch the tube at an outer curve of a bend and to butt or compress it at an inner curve of a bend.

## Description

The invention relates to a tube, which will not kink during normal use*.*

### Brief Description of the Drawing

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figures 1A, 1B illustrate what happens, when a tube is subjected to kink.
Figures 2A, 2B, 2C illustrate a side view, a cross-sectional view and another side view of a tube with corrugations.
Figure 3 illustrates a photo of tube with corrugations.
Figure 4 illustrates a photo of a tube with corrugations and how kink will not happen, even if the tube is bent sharply.
Figures 5A to 5D illustrate a tube, where the corrugations initially extend in one direction only.
Figures 6A, 6B illustrate a side view and a cross sectional view of a tube where the tube material is longer than the un-stretchable support.
Figure 7 illustrates a cross-sectional view of a tube with corrugations.

### Detailed Description

Embodiments relate to a tube having a first end and a second end, wherein a longitudinal direction is defined as extending from the first end to the second end, and a transverse direction is defined as transverse to the longitudinal direction, wherein the tube comprises corrugations in the longitudinal direction, characterised in that the tube has at least one longitudinally extending support configured for preventing stretching in the longitudinal direction.

Embodiments relate to a tube having a first end and a second end, and a first length of the tube extends from the first end to the second end, wherein the tube comprises at least one non-stretchable support corresponding to the first length, the tube further comprises material constituting the lumen of the tube, which material has a second length, that is longer than the first length.

A tube as described above has the advantage that it is very flexible in a transverse direction, so that it is easily bendable, but at the same time it is un-stretchable in a longitudinal direction. Furthermore, it is possible to stretch the tube in an outer curve of a bend and to butt or compress it in an inner curve of the bend thereby keeping a cross-section across the apex of the bend open for liquid to pass through. This has the effect that the tube will be very unlikely to kink even if it is folded onto itself or curled up in the longitudinal direction.

The longitudinal direction is the direction from one end of the tube to the other end. The transverse direction is the direction perpendicular to the longitudinal direction, which corresponds to the direction across the tube.

An irrigation system typically comprises a reservoir or container for irrigation liquid, an anal probe and tubing connecting those two. The system may also include a pump for pumping the irrigation liquid into the intestines. A tube as described herein may be suitable for use in such a system.

A tube as described herein may also be used in connection with urine collection. In systems for urine collection, a user may be provided with either a urinary catheter or a urisheath which leads the urine from the bladder. A urisheath is typically provided with a connector part at its end, to which a connector on a tube can be connected. The tube will then in the other end be connected to a collecting bag. Similar to that, a catheter is also typically provided with a connector part at its end, to which a connector on a tube can be connected. And again, the tube may in the other end be connected to a collecting bag. In case an intermittent urinary catheter is used, the tube may not be connected to a collecting bag, but may rather be provided with an outlet, which is configured for leading the urine directly into a toilet. In this case, the tube may function as an extension tube for an intermittent catheter. For an indwelling catheter, the tube will typically be connected to a collecting bag.

In the context of this description, a tube is considered to be an element having a longitudinal extension and which is able to allow liquid to flow through it. A cross-section of the tube may have any shape - and even be closed prior to use.

In this context, kink is defined as an unintended closure of the tube across a diameter, typically due to a bend in the longitudinal direction. When a user handles a rather long tube (e.g. more than 50 cm long), it is easy to inadvertently bend the tube in a longitudinal direction. If such a bend is sharp, then there is a risk that the tube kinks and thereby closes off the inner lumen at the apex of the sharp bend.

The tube as described herein, will not kink and this means that a cross-section across the apex of the bend will always be open as liquid flows through it. So even if the tube is bent sharply, e.g. folded in a longitudinal direction, the tube will not be closed at a cross-section across the apex of the bend.

The apex of the bend will have an outer curve where the material stretches and an inner curve where the material butts.

In the context of this disclosure, a non-stretchable support is defined as a structure which is able to substantially prevent stretching in a longitudinal direction. By substantially prevent is meant that it will not stretch more than 10 % during normal use.

In an embodiment, the tube has two longitudinal supports.

In an embodiment, the supports are placed 180 degrees from each other in a circumferential direction around the tube.

In an embodiment, the tube is made of thin film or foil material. By thin material is meant a material which is below 90 mu, e.g. below 50 mu or below 20 mu or even below 10 mu. It is contemplated that it may be as thin as 7 mu.

Examples of materials which may be useful are Polyethylene (PE), Polypropylene (PP), Polyethylene terephthalate (PET) or Ethylene Vinyl Acetate (EVA).

These materials may be used either as newly processed materials or as recycled materials. Embodiments relate to the tube being made at least partly of recycled materials. PET is very common as a recycled material and this may form at least a part of the material in the tube.

EVA is a material, which is easily stretchable, so this may be easy to process in a vacuum forming process.

Other examples of material are materials including metal, e.g. aluminium, or rubber-materials.

The materials may be used as single-layer or multilayer film or foil-materials and may be combined with each other so as to obtain the desired thickness and flexibility. For example, aluminium may form part as a thin layer in combination with PE or EVA or combinations of PE and EVA and aluminium in a very thin layer. Likewise, recycled PET may form a layer in combination with a more flexible material, for example a layer of EVA or low-density PE.

In an embodiment, the support is made by welding the tube in the longitudinal direction. This means that the tube has one or two longitudinal welds along its length. This is an easy way to create a non-stretchable support in a longitudinal direction. In embodiments the support comprises three or four welds. The welds may be positioned equidistantly around the circumference but could also be positioned such that a first and second weld are positioned with e.g. 60 degrees between them followed by 120 degrees to the third weld and again 60 degrees to the fourth weld. This leaves again 120 degrees between the fourth and first weld.

In an embodiment, the tube has a first width in the transverse direction, which is larger than a second width of the tube in the transverse direction, wherein the first and second widths alternate in a longitudinal direction.

In an embodiment, the tube has a lumen with an interior surface and an exterior surface, wherein the longitudinal support extends radially beyond the exterior surface of the lumen.

Embodiments relate to a tube with a storage configuration, in which an inner lumen of the tube is substantially closed along the length of the tube. The tube has a use configuration, in which the inner lumen is open to allow flow of liquid through it.

The transformation from the storage configuration to the use configuration may be done by simply connection the tube to a liquid supply. The pressure from the liquid will automatically open the inner lumen of the tube.

By substantially closed is meant that the inner lumen has a volume in a storage configuration of less than 10 % of the volume of the inner lumen in a use configuration.

Such an air-free tube is advantageous in use in an irrigation system, because it is undesirable to pump air into the bowels of a user as the irrigation process is initiated. Therefore, in other systems, where the tube includes a volume of air, the user might have to prime the system, i.e. wait for liquid to have pushed the air out of the tube, prior to initiating the irrigation process.

Embodiments relate to the tube having a cross-sectional circumference in which a first part around the circumference is convex and a second part around the circumference is concave. The concave part of the circumference extends into the convex part, such that the two parts are close together.

Embodiments relate to a tube comprising a first and a second half in the longitudinal direction, wherein each first and second half has corrugations in the longitudinal direction and the tube in a storage configuration is collapsed such that the corrugations of the second half is collapsed into the corrugations of the first half.

In an embodiment, the tube is configured for use in an anal irrigation system.

In an embodiment, the tube is configured for functioning as a connection tube connecting a reservoir bag and a urinary catheter.

In an embodiment, the tube is configured for functioning as a connection tube connecting a reservoir bag and a urisheath.

In an embodiment, the tube is configured for functioning as an extension tube for a urinary catheter.

Particular in uses relating to anal irrigation or with urine drainage, it is an advantage if the tube is free of kink. For example, in relation with an anal irrigation procedure, a user might risk that the liquid is prevented from entering the bowels, if the tube kinks. In relation with urine collecting, a leakage might occur, if the tube kinks in such a way that the urine is prevented from being drained into a collecting bag.

Embodiments relate to a tube where the tube comprises a connector in one end. The tube may also have a connector in both ends.

The connector may be simple friction fit luer-connector configured for connecting to another luer-connector. It may also be a bayonet connector or a threaded connector.

Embodiments relate to a tube which is configured for being used only once. In other words, the tube is configured for one-time use. In this context, configured for one-time use means that the tube is made of material, which does not withstand being rinsed or otherwise cleaned. This means that the tube may be made of thin recycled material and can be discarded for further recycle following use. This saves material and also relieves the user of a tiresome process of cleaning the tubes and leave them to dry.

### Detailed Description of the Drawing

Embodiments, and features of the various exemplary embodiments described in this application, may be combined with each other ("mixed and matched"), unless specifically noted otherwise.

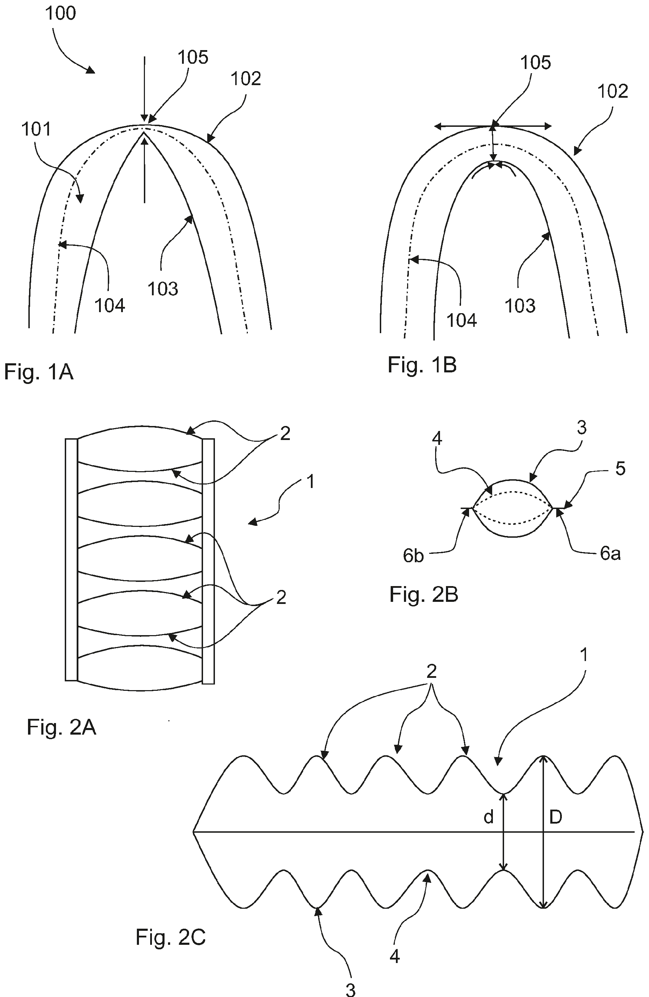

Figures 1A, 1B illustrate the problem arising due to kink. In figure 1A, a tube 100 is shown. The tube is made of film material 101. When it is bent, the tube will have an outer curve 102 and an inner curve 103 in the bend. A centreline 104 is also indicated in the drawing. The kink occurs, because the inner curve collapses in cusp bend and thereby the cross-section of tube is (almost) closed as indicated at the arrows 105. In figure 1B, the situation is different. The outer curve 102 of the tube is able to stretch as indicated at 105 and the inner curve 103 will compress or butt as indicated at the arrows. This has the effect that the cross-section at the bend remains open.

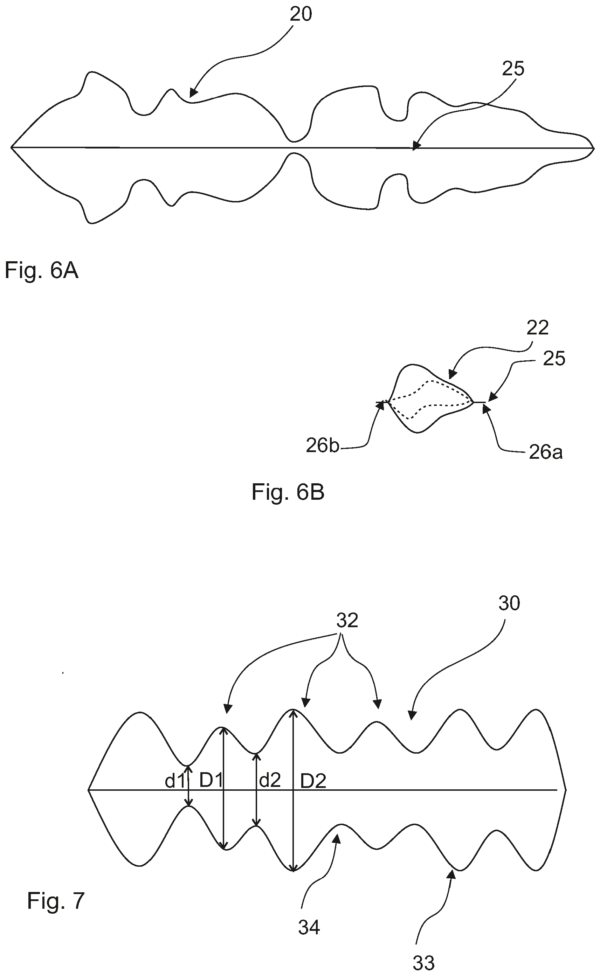

Figures 2A, 2B, 2C illustrate a tube having the ability to stretch in an outer curve and compress or butt in an inner curve. Figure 2A shows a tube seen from a side, figure 2B shows a cross-sectional view of the same tube and figure 2C shows the same tube seen from a side perpendicular to figure 2A. The tube 1 is made of rather thin material and is corrugated in a longitudinal direction. In figure 2C, the corrugations 2 are seen from a side view, so that it is possible to see that a cross-sectional diameter alternates between a smaller diameter d and a larger diameter D in the longitudinal direction. In the illustrated embodiment, the smaller diameter d and the larger diameter D are shown as being consistent through-out the length of the tube. However, embodiments may be made, where the smaller diameter and/or the larger diameter D varies along the length. An example is shown in figure 7.

Figure 2B illustrates a cross-sectional view of the tube 1. In this figure, it is possible to see that the tube is made of rather thin film-material. Furthermore, the corrugations 2 are illustrated as a top 3 and a trough 4, where the trough 4 is shown in a dotted line indicating that it is not in the same cross-section as a top. The top 3 and the trough 4 can also be seen in figure 2C. In this illustration, it can be seen that the larger diameter D is at a top and the smaller diameter d is at a trough.

In figures 2A, 2B, 2C, the non-stretchable support 5 is also illustrated. In this embodiment, the non-stretchable support is in the form of two welds 6a, 6b, one on each side of the tube in the longitudinal direction. This means that the two supports in this embodiment are placed 180 degrees apart in a cross-sectional view of the tube, figure 2B.

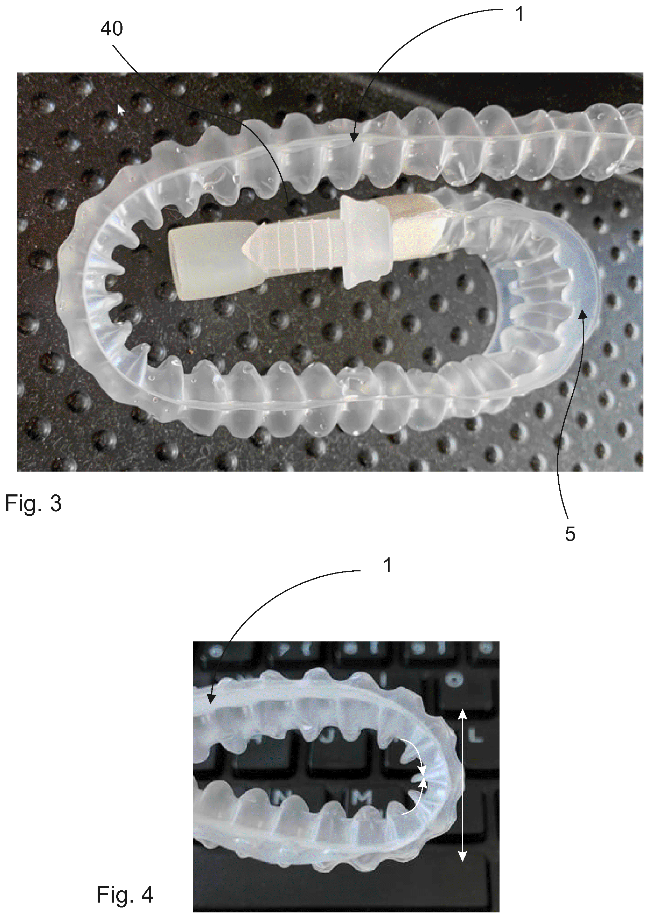

Figures 3 and 4 show photos of a tube 1. In figure 3, the un-stretchable support 5 is illustrated.

In figure 4, it is illustrated that even if the tube is bend sharply, the tube will stretch at an outer curve and butt at an inner curve. This is in this embodiment made possible by the combination of the corrugated tube and the non-stretchable support.

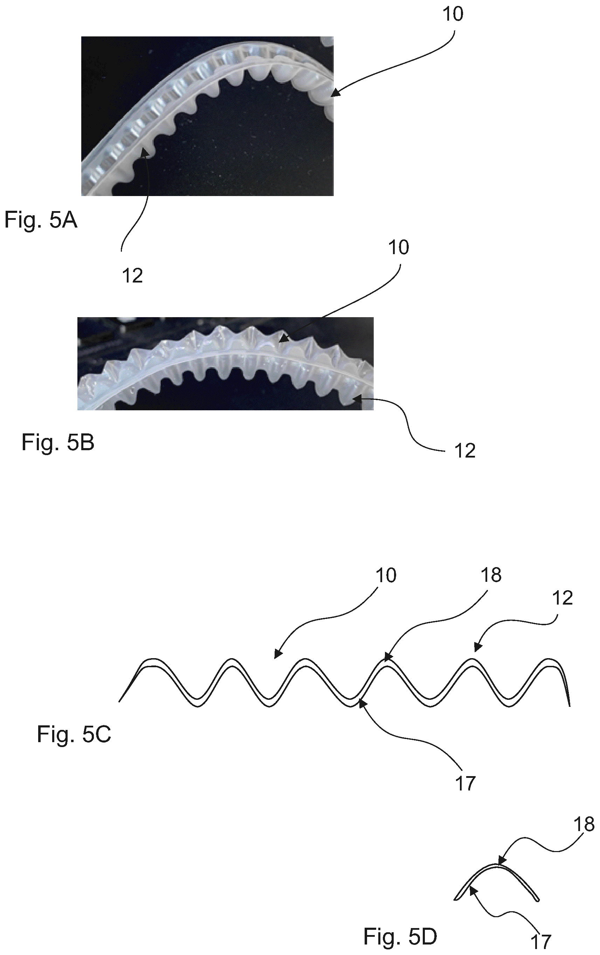

Figures 5A, 5B, 5C and 5D illustrate a tube 10 which is manufactured such that the corrugations 12 are made only to one side initially, i.e. such that the corrugations 12 of one half of the tube 17 is collapsed into the corrugations of the other half of the tube 18. This means that the tube has two film layers with very little (or no) air between them prior to use, see figure 5A. When the tube is used, i.e. is pressurised by flow of liquid, the tube will un-fold itself as in figure 5B, where the corrugations 12 to both sides can be seen. In figure 5C, a cross section in a longitudinal direction is seen. The two halves 17, 18 can be seen facing in the same direction such that one of the halves 17 is collapsed into the half 18. In the half 18, the corrugations are convex, and in the half 17, the corrugations are concave.

Figures 6A, 6B illustrate a tube 20 where the tube material 22 is longer than the un-stretchable support 25. For the corrugated tubes in figures 2 to 5 the tube material is also longer than the un-stretchable support, but in figures 6A, 6B, the longer tube material is in a random way. By random is meant that the tube 20 is not corrugated, but instead the tube material 22 is welded along its length to provide a non-stretchable support 25 in such a way that a length of the tube material in a longitudinal direction is longer than the un-stretchable support 25 in the same longitudinal direction. The non-stretchable support 25 consists of two welds 26a, 26b positioned opposite each other, i.e. with 180 degrees between around the circumference of the tube, see figure 6b.

Figure 7 illustrates a longitudinal cross-section of a tube 30 in which the corrugations 32 varies along its length. As illustrated, the cross section varies between the tops 33 and the troughs 34 from a first small diameter d1 to a first larger diameter D1 back to a second smaller diameter d2 and then to second larger diameter D2 and so forth.

## Claims

1. A tube having a first end and a second end, where a first length of the tube extends from the first end to the second end, wherein the tube comprises at least one longitudinal extending support configured for preventing stretching in the longitudinal direction, the support having a length corresponding to the first length, wherein the tube further comprises material constituting the lumen of the tube, which material has a second length, that is longer than the first length, wherein the tube is configured for being stretched in an outer curve of a bend and to butt or compress in an inner curve of the bend thereby keeping a cross-section across the apex of the bend open for liquid to pass through.

2. The tube as claimed in claim 1, wherein the tube has two longitudinal supports.

3. The tube as claimed in any of the preceding claims, wherein the tube is made of thin film or foil material.

4. The tube as claimed in any of the preceding claims, wherein the tube has a storage configuration, in which an inner lumen of the tube is substantially closed along the length of the tube and wherein the tube has a use configuration, in which the inner lumen is open to allow flow of liquid through it.

5. The tube as claimed in claim 4, wherein the tube comprises a first and a second half in the longitudinal direction, wherein each first and second half has corrugations in the longitudinal direction and the tube in the storage configuration is collapsed such that the corrugations of the second half is collapsed into the corrugations of the first half.

6. The tube as claimed in claim 4, wherein the tube comprises a cross-sectional circumference in which a first part around the circumference is convex and a second part around the circumference is concave.

7. The tube as claimed in any of the preceding claims, wherein the support(s) is made by welding the tube in the longitudinal direction.

8. The tube as claimed in any of the preceding claims, wherein a first width of the tube in the transverse direction is larger than a second width of the tube in the transverse direction, wherein the first and second widths alternate in a longitudinal direction.

9. The tube as claimed in any of the preceding claims, wherein the tube is configured for functioning as a connection tube connecting a reservoir bag and an anal probe in an anal irrigation system.

10. The tube as claimed in any of the preceding claims, wherein the tube is configured for functioning as a connection tube connecting a reservoir bag and a urinary catheter.

11. The tube as claimed in any of the preceding claims, wherein the tube is configured for functioning as a connection tube connecting a reservoir bag and a urisheath.

12. The tube as claimed in any of the preceding claims, wherein the tube is configured for functioning as an extension tube for a urinary catheter.

13. The tube as claimed in any of the preceding claims, wherein the tube is provided with a connector in one or both ends.

14. The tube as claimed in any of the preceding claims, wherein the tube is configured for one-time use.
